# EUROPEAN PATENT APPLICATION

(11) **EP 0 892 046 A2**
(43) Date of publication of application: **20.01.1999**
(21) Application number: 98111352.5
(22) Date of filing: 19.06.1998
(51) Int. Cl.: C12N 15/12, C07K 14/72, C07K 16/28, C12N 5/16, G01N 33/68

(54) **Melatonin-receptor expression cells and their uses**

(30) Priority: 19.06.1997 JP 180537/97
(71) Applicant: JCR PHARMACEUTICALS Co., LTD., Ashiya, Hyogo 659 (JP)
(72) Inventor: Yokoyama, Tetsuo, Kobe, Hyogo (JP); Shirono, Hiroyuki, Kobe, Hyogo (JP); Koga, Jun-ichi, Kobe, Hyogo (JP)
(74) Representative: Keller, Günter, Dr.

(57) **Abstract**

The present invention provides materials capable of screening human melatonin which comprise an animal cell containing an expression plasmid for the gene encoding a human melatonin receptor protein and having said protein expressed therein, as well as a method for screening the same, thus permitting not only human melatonin but also hormones showing affinity for the same, and their agonists or antagonists to be screened with an enhanced degree of precision.

## Description

The present invention relates to preparation of animal cells capable of expressing a gene encoding a human melatonin receptor protein, to a method for screening a hormone compound showing affinity for the said protein and its agonist or antagonist, and to uses of a recombinant human melatonin receptor protein produced according to the present invention.

Melatonin, a hormone secreted by the pineal gland of the brain, occurs in body fluids, such as cerebrospinal fluid and blood, and its production is known to undergo marked circadian changes [L. Wetterberg et al., Journal of Neural Transmission. Suppl. 13, 289-310 (1978)]. The mean blood level of melatonin is about 63 pg/ml for males and about 100 pg/ml for females, respectively, and increases by 50 to 100 fold during night than in the daytime.

Melatonin is biosynthesized from tryptophan as a lead compound by enzymatic modification through N-acetylserotonin via serotonin. Biosynthesis of melatonin shows a circadian rhythm, since M-acetyltransferase acting to catalyze acetylation of serotonin exhibits a circadian rhythm. In the brain tissues, there exist a variety of bioamines, such as catecholamine, adrenalin and serotonin, which are produced through modification of the corresponding amino acids and act as a neurotransmitter. It has been clarified that melatonin, when administered to humans, causes phase changes in circadian rhythm, and actually, the reports were published that melatonin is effecive for the treatment of circadian rhythm disorders inclusive of jet lag syndrome.

Melatonin has heretofore been determined quantitatively mainly by means of radioimmunoassay (RIA). Because melatonin shows a lowered molecular weight and there occur naturally a large number of its homologs, however, it is quite difficult to produce highly specific antibodies against melatonin. Moreover, melatonin is present not only in animals but also in plants and cannot be used directly for immunization, as this does neither bring about any antigen-antibody reaction nor produce antibodies. In light of the fact that melatonin does not have any highly reactive functional group, therefore, antibodies are produced through immunization with melatonin linked to a carrier.

Recently, a melatonin receptor was cloned [Ebisawa et al., Proc. Natl. Acad. Sci. U.S.A., 91, 6133-6137, (1994)], and as a result, the melatonin receptor was found to belong to a seven membrane-spanning, G protein coupled type of receptors. Among the melatonin receptors, mel-1a localizes in the hypothalamic suprachiasmatic nuclei, while mel-1b exists locally in the retina, and both of these receptors exhibit high specificity and affinity for melatonin, with their IC₅₀ values being at 10⁻⁹ M [Steven M. Reppert et al., Neuron, 13, 1177-1185 (1994), Steven R. Reppert et al., Proc. Natl. Acad. Sci. U.S.A., 92, 8734-8738 (1995)]. Referring to melatonin receptors of origins other than mammalians, mel-1c was also cloned.

The nuclear receptors for melatonin, which constitute a transcription factor, control the transcription activities of the downstream genes, and one of the known genes which are controlled by such receptors has been found to be the gene for 5-lipooxygenase [Dieter Steinhilbert et al., J. Biol. Chem., 270, 7037-7040, (1994)].

On the other hand, the membrane receptors for melatonin belong to the G-protein coupled receptor family, and upon melatonin binding, have their trimeric G proteins undergo dissociation into α-subunit and βγ-subunit, which subunits individually in turn act as a second messenger to cause downstream signal transduction [Paula A. Witt-Enderby et al., Molecular Pharmacology, 50, 166-174 (1996)].

As is described above, melatonin and its receptors play an important role in the intracellular information transmission function.

Sleep-awakening action and brain hormone secretion are known to exhibit a definite rhythm, which reveals that sleep changes or sleep disorders are closely connected with such hormone secretion. Melatonin is presumed to affect stresses and aging, as well as the immune or endocrine system, but there has not been established so far any high-precision technique for quantitative determination of melatonin.

The present invention provides the use of melatonin receptors expressed in animal cells in the functional analysis of melatonin-affinity substances, hormones, and their agonists or antagonists.

The melatonin receptors occur in three subtypes, as described in the above; two kinds of the melatonin receptors, mel-1a and mel-1b, were discovered in mammalians inclusive of humans. By way of an example, the present inventors conducted screening of the gene base sequence of the melatonin receptor mel-1a by use of the gene data base (supplied by Gen Bank Inc. of U.S.A.) and constructed the primers for cloning and for sequencing.

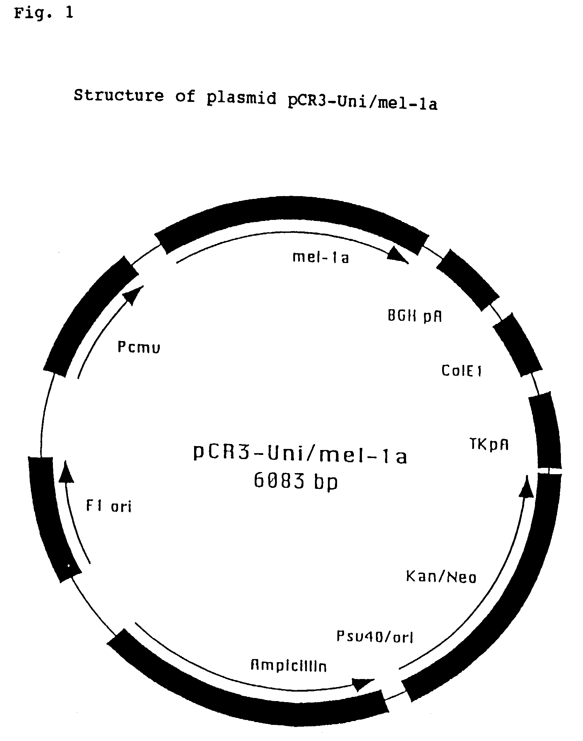

The base sequence for mel-1a was obtained by amplifying poly A+RNA from the human whole brain, hypothalamus and fetal brain by the RT-PCR, followed by the two-step PCR method with use of the semi-nested PCR method (refer to Figs. 1, 2 and 3).

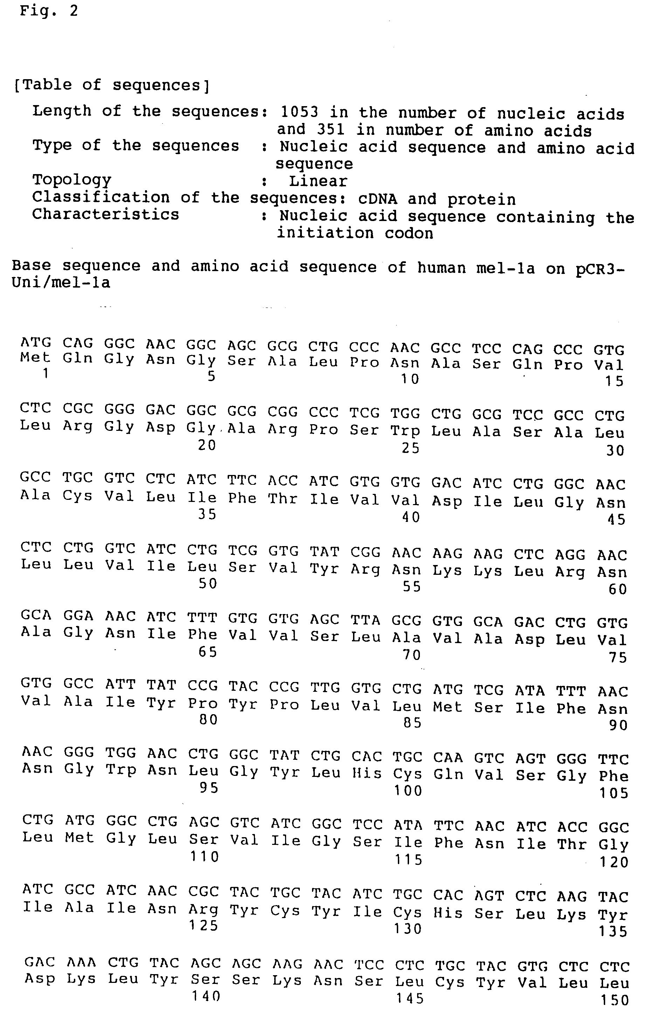

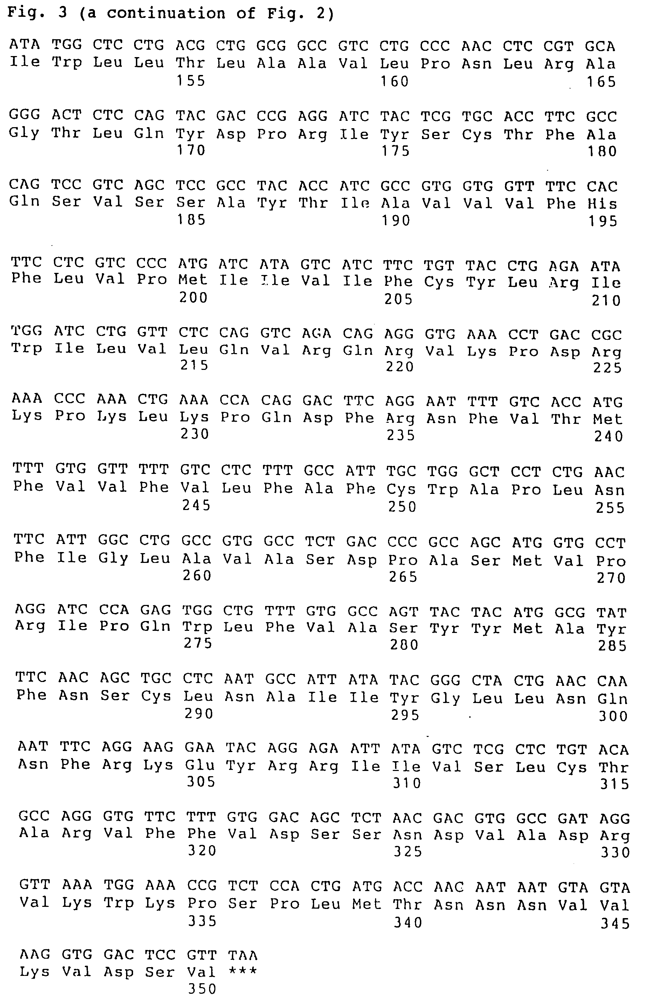

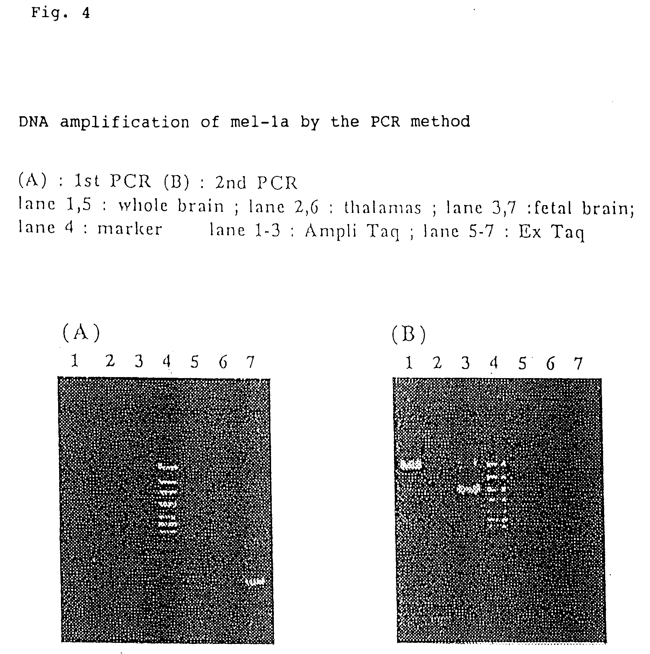

The PCR product of the human whole brain and fetal brain was linked to plasmid pCR3-Uni, followed by insertion into the competent cells to produce the transformed cells. All of the clones were subjected to colony PCR analysis, whereby the least mutated clones alone were picked up by restriction enzyme analysis (fig. 4). The resultant clone was observed to have adenine undergo mutation to guanine at the position 90 of the base sequence (Figs. 2, 3. 5 and 6), but to show no mutation in the amino acid (Leu) encoded by the said base.

cDNA for the melatonin receptor gene may be produced using a variety of the known techniques. Different DNA fragments obtained from cDNAs for the melatonin receptors, chemically synthesized DNAs and genomic DNA gene sequences can be joined to produce a hybrid-like melatonin receptor gene. During the course of this step, the known techniques can also be utilized to facilitate mutation to be caused in the melatonin receptor gene, as may be exemplified by various derivatives which may be provided by subjecting the amino acids and peptides to replacement, deletion or addition while retaining substantially the activity of the melatonin receptor protein, and therefore such mutant receptor genes and mutant receptor proteins are included in the present invention, as well.

The present inventors picked up the clone having a replacement in the base sequence mentioned above but showing no mutation in the amino acid sequence and joined the same to a plasmid, followed by insertion into Escherichia coli and cultivation to thereby prepare in large quantities the expression plasmid pCR3-Uni containing the mel-1a clone. The expression plasmids which can also be used include pA1-11, pXT1, pRc/CMV, etc., while the promoter may be exemplified by SV40 promoter, LTR promoter and CMV promoter.

For the promoter, furthermore, there can be employed those containing enhancers, selection markers, SV 40 reproduction origins, etc. In cases where transient expression or stable expression is desired, such expression becomes feasible by selection of the episome factor in the viral sequence.

The present inventors produced the transformed cell by transfecting the prepared expression plasmid (mel-1a/pCR3-Uni) into the CHO cell, a mammalian cell, by the calcium phosphate method. For the production of such transformed cell, there may be used other mammalian cells, such as COS-1 cell, LTK cell and NIH3t3 cell. The transformed cell was cultivated and selected for the neomycin resistance to produce the cell having mel-1a receptor gene expressed therein. The present invention includes the cells having the melatonin receptor gene expressed therein.

Referring to the cell selection markers, it may be possible to utilize ampicillin resistant gene (Amp') and neomycin resistant gene (G418 resistance) as well as dihydrofolate reductase gene (methotrexate resistance), etc.

By following the known procedures, the cell having the human melatonin receptor gene expressed therein can be cultivated, harvested, then subjected to cell and cell-membrane disruption by means of freezing/thawing, homogenizer or surfactants, and freed of cell debris by the centrifugation procedure to thereby produce a solution containing the human melatonin receptor protein.

Moreover, such receptor protein can be purified by any arbitrary combinations of the known purificatory techniques, such as salting out, ion exchange chromatography, gel permeation chromatography, affinity chromatography, normal-phase and reverse-phase high performance liquid chromatography and SDS-electrophoresis.

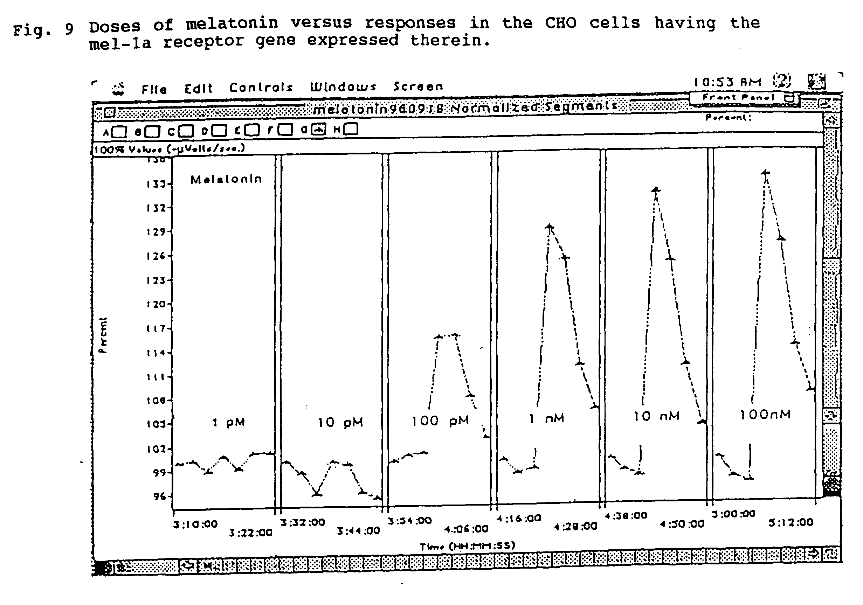

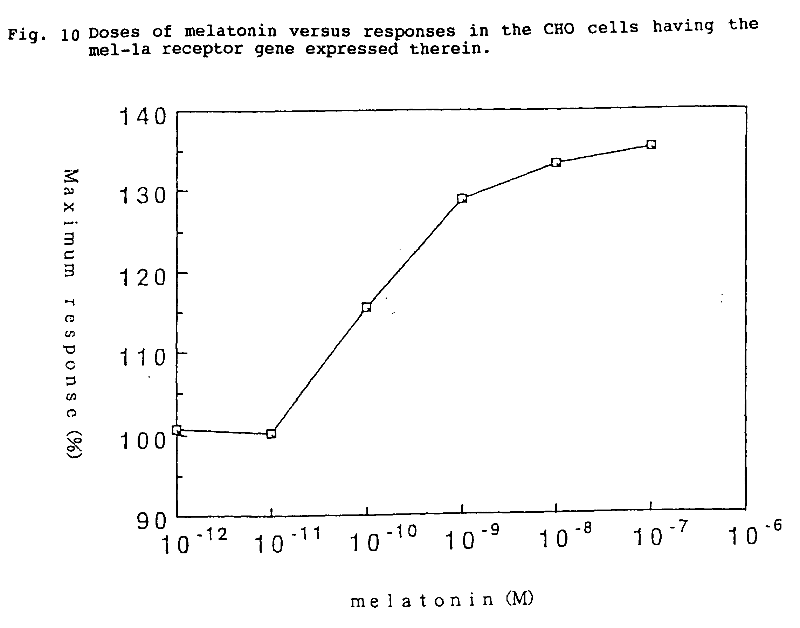

Then, response and quantitative determination of melatonin were attempted with the CHO cell having mel-1a expressed using a cytosensor. As a result, the metabolic rate was noted to rise in a dose-dependent manner at concentrations of melatonin in the range of 100 pM to 100 nM (refer to Figs. 9 and 10), with the metabolic activity after addition of 100 nM of melatonin being shown to increase up to about 1.5 times greater than under normal conditions.

Melatonin stimulation was transient; actually, the metabolic activity declined about 40 minutes after the stimulation, but was noted to restore to a similar level after repeated stimulation. Additionally, the specificity of the melatonin receptor was investigated; even addition of its homologs, tryptophan, 5-methoxytryptophan, 5-methoxytryptamine, serotonin and N-acetylserotonin was found not to bring about an increase in metabolic rate via the receptor, suggesting that the cell having the melatonin receptor gene expressed therein is specific to melatonin (refer to Figs. 7 and 8). In this manner, the quantitative determination method with simultaneous use of the cell having the melatonin receptor gene expressed therein and a cytosensor excels in functional assaying performance to the conventional RIA method (refer to Fig. 11).

The present invention includes uses of the cell having the melatonin receptor gene expressed therein in a method for screening hormones for the receptor, and its synthetic and natural agonists or antagonists. The production of polyclonal antibodies and monoclonal antibodies for the melatonin receptor proteins is easy and ready to be realized by using the known techniques, and is included in the scope of the present invention.

The following examples and figures are intended to illustrate in detail the present invention, and the present invention is in no way restricted to them.

### Brief Description of Drawings

Fig. 1 is a gene mapping of the plasmid in which pCR3-Uni was used as a vector in order to express the mel-1a receptor in the CHO cells. Pcmv and Kan/Neo designate the expression CMV promoter and kanamycin/neomycin resistant gene, respectively.
Fig. 2, together with Fig. 3, shows the base sequence and amino acid sequence for the mel-1a receptor having pCR3-Uni vector inserted therein. Unlike the known base sequences, the base sequence has adenine at the 90th position mutated to guanine, but there has not been observed any mutation replacement for the amino acid (leucine) encoded by the base.
Fig. 3 is a continuation of Fig. 2.
Fig. 4 illustrates the electrophoresis patterns for the PCR product in Example 1 (4), wherein (A) and (B) designate the patterns for the 1st PCR and 2nd PCR, respectively.

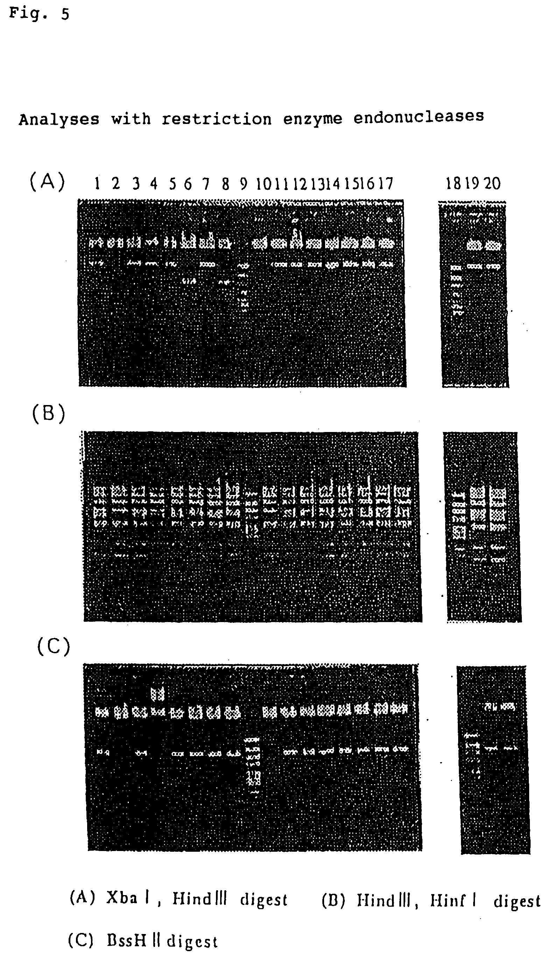

Fig. 5 illustrates the electrophoresis patterns recorded in the restriction enzyme analyses in Example 2 (2), wherein (A), (B) and (C) designate the enzyme treatments with XbaI and HindIII, HindIII and HinfI, and BssHII, respectively.

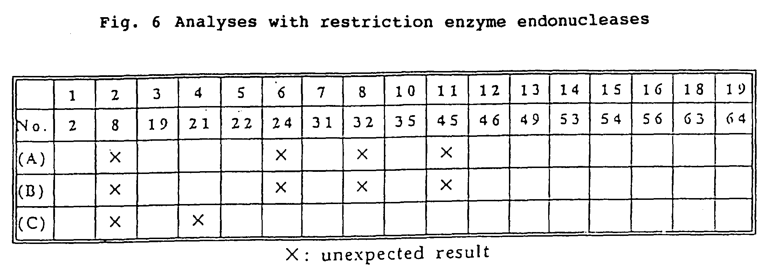

Fig. 6 tabulates the results of the restriction enzyme analyses in Example 2 (2), wherein (A), (B) and (C) are the same as defined in Fig. 5, and the symbol "X" designates the clones in which the objective molecular weight was not able to be detected by the restriction enzyme treatment.

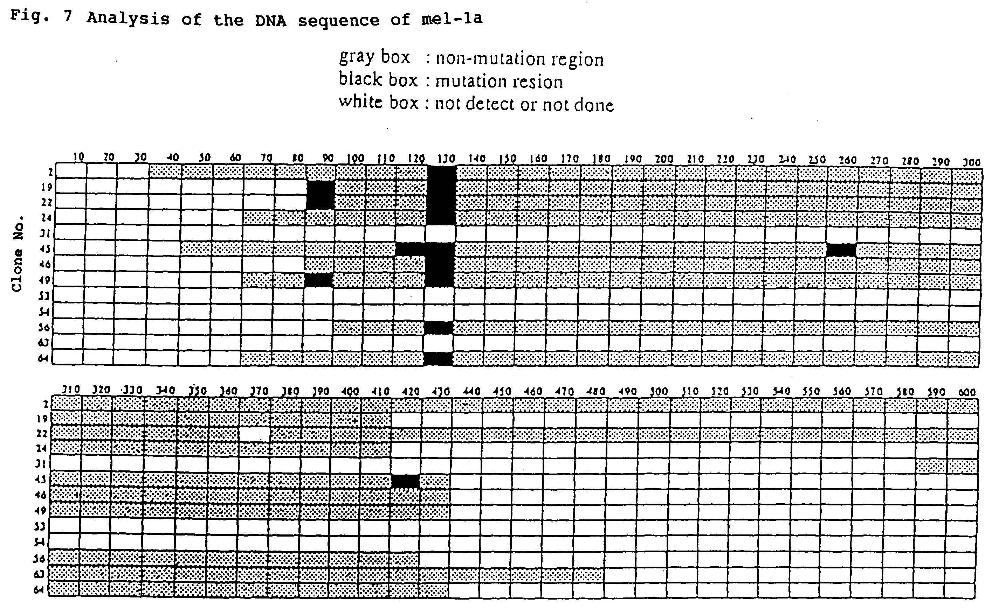

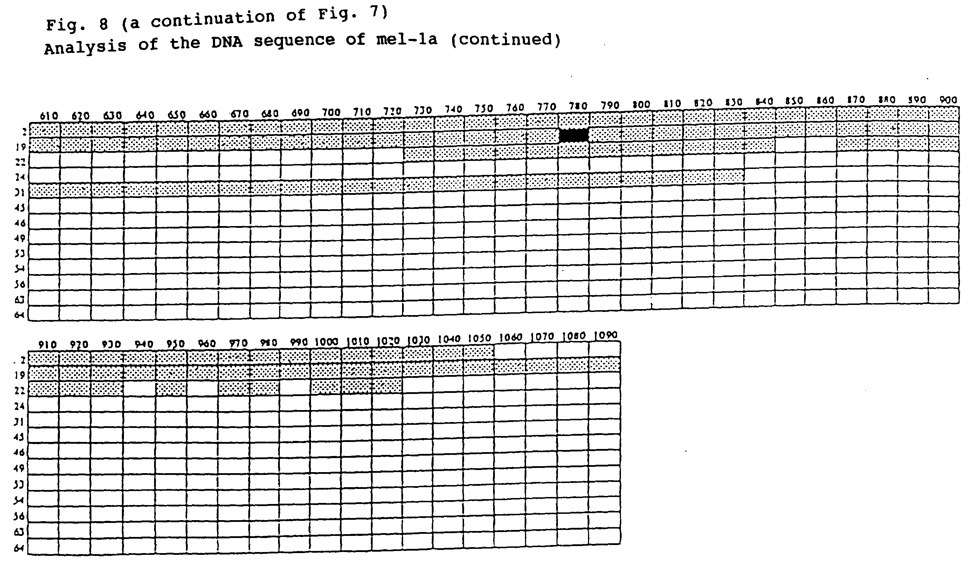

Fig. 7, together with Fig. 6, shows the DNA sequencing analysis for the clones found normal in the restriction enzyme treatment, wherein the symbols "black square" and "gray square" designate a site having undergone mutation and a site freed of mutation, respectively, with the symbol "white square" meaning an unanalyzed site.
Fig. 8 is a continuation of Fig. 7.
Fig. 9 is a graph showing the results of analysis of the mel-1a receptor expressed cell with the cytosensor in Example 3 (3), from which it is evident that the metabolic activity of the CHOP cells rises in a dose-dependent manner at the melatonin concentrations of 100 pM or more.
Fig. 10 is a dose-dependent saturation curve obtained by plotting the maxima of the metabolic activity against melatonin added at different concentrations in Fig. 9.

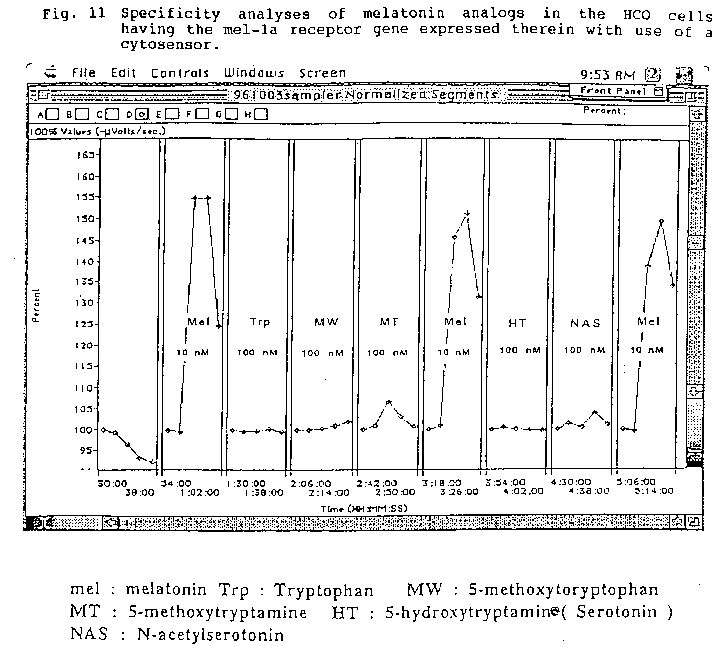

Fig. 11 is a graph showing the responsiveness to the mel-1a receptor of melatonin and its homologs as determined by the cytosensor, wherein the symbols "Mel", "Trp", "MW", "MT", "HT", and "NAS" designate melatonin, tryptophan, methoxytryptophan, methoxy-tryptamin, 5-hdyroxytryptamin and N-acetylserotonin, respectively.

### Examples

### Example 1: PCR Cloning of mel-1a

### (1) Preparation of primers

The gene nucleotide sequence for mel-1a was screened on the basis of the Inter-Net data base (Gen bank), and the following sequences were designed and used as primers:

### Primer for cloning

mel-1a-FF; 3'-ATGGCCCTGCGGCCGGGACGCGAAC-5'
mel-1a-F ; 3'-GACCATGCAGGGCAACGGCAGCGC-5'
mel-1a-R ; 3'-TTAAACGGAGTCCACCTTTACTAC-5'

### Primer for sequencing

mel-1a-350F; 3'-TATCTGCACTGCCAAGTCAGTGGGTTCCTG-5'
mel-1a-700F; 3'-GGTTCTCCAGGTCAGACAGAGGGTGAAACC-5'

### (2) Synthesis of cDNA of the 1st-strand

Each polyA+RNA was admixed with 33µl of DFEC treated water, followed by incubation at 65°C for 5 minutes and then at 37°C for 5 minutes. The solution of polyA+RNA was added to the 1st-strand cDNA Kil incubated in advance at 37°C for 5 minutes, and the solution mixture was incubated for 5 minutes, admixed slightly with tube and centrifuged quickly, followed by further incubation at 37°C for 60 minutes.

### (3) Phosohorylation of forward-directing primer

As reagents, 10-fold diluted T4 Polynuleotide Kinase Buffer (produced by Takara) (1 µl), 10 mM ATP (1 µl), T4 Polynucleotide Kinase (15 U/µl), and 100 µM forward-directing mel-1a primer (7 µl) were added to a microtube, followed by incubation at 37°C for 1 hour. Further incubation was effected at 95°C for 10 minutes to inactivate T4 Polynucleotide Kinase.

### (4) PCR and ligation reaction of mel-1a, and transformation of Escherichia coli

As reagents, 10-fold diluted Reaction Buffer (Note 1) (3 µl), 2.5 mM dNTP (9.6 µl), phosphorylated forward-directing mel-1a primer (2.1 µl), 100 µM reverse mel-1a primer (2.1 µl), Tag DNA polymerase (0.3 µl), 1st strand cDNA or 1st PCR product (3 µl) and H₂O (36.9 µl) were added to a microtube, followed by 35 cycles of the 1st PCR reaction at 94°C for 1 min., at 60°C for 1 min. and at 72°C for 2 min. The PCR product was subjected to electrophoresis, stained with ethydium bromide and photographed under UV irradiation (Fig. 4).
Note 1: Buffer as attached to Amplitag DNA polymerase (Perkin Elmer Co.)

Since mel-1a protein is present locally on the hypothalamus, mRNA from the hypothalamus is required to amplify DNA originated from mel-1a by RT-PCR. The hypothalamus of a human origin is difficult to be obtained, and the amplification by RT-PCR was made with use of mRNA from the whole brain, thalamus and fetal brain.

In the first place, the primers mel-1a-FF and mel-1a-R, were used to perform the amplification by PCR (1st PCR) (Fig. 4A). However, the desired DNA band was minute and difficult to be detected, and consequently, the amplification by PCR was effected with the primers mel-1a-F and mel-1a-R, while using the 1st PCR as a template, to thereby detect the desired DNA band from the whole brain (2nd PCR) (Fig. 4B). From the fetal brain, there was also detected the specific DNA band, which however showed a slightly lowered molecular weight than the desired gene. As the polymerase for PCR, Ampli Tag (Perkin Elmer) and Ex Tag (Takara) were used for the lanes 1 to 3 and lanes 5 to 7, respectively, with the result that the amplification was noted with Ampli Tag.

The desired band was cut out and purified with use of Gene Clean 11. A DNA solution (5 µl) containing about 10 ng of the resultant purified mel-1a and pCR-Uni (note 2) (1 µl) containing 30 ng of plasmid were subjected to incubation treatment at 14°C overnight using Ligation Kit ver. 2 (sol. I)(6 µl) to produce recombinant plasmid (mel-1a/pCR3-Uni) (Fig. 1). TOP10F' competent cells (Note 3) were dissolved over an ice bath and distributed in 50 µl portion into a Falcon tube, to which 2 µl of 0.5M mercaptoethanol solution was added, followed by gently mixing over an ice bath. Furthermore, 5 µl of the ligation product, mel-1a/pCR3-Uni, was added, followed by incubation for 30 min. over an ice bath.

A solution of Escherichia coli was incubated at 42°C for 30 sec. and then cooled for 2 min. over an ice bath, and 450 µl of SOC medium incubated in advance at 37°C was added, followed by shake culture at 37°C for 1 hr.
Note 2: Eukaryo TA Cloning Kit-Unidirectional (Invitrogen Co.).
Note 3: Ibid.

### Example 2 Analysis of clones

### (1) Colony PCR

A culture broth was seeded into a LB plate containing 50 µl/ml of ampicillin, followed by cultivation at 37°C overnight, and a small amount of the ampicillin resistant *Escherichia coli* colony was harvested and dissolved in a Microtube containing 1 µl of 10% Triton X-100. Added to this Microtube were 10-fold diluted reaction buffer (3 µl), 2.5 mM dNTP mixture (3.2 µl), phosphorylated forward-directing mel-1a primer (1.4 µl), 100 µl/ml reverse-directing mel-1a primer (1.4 µl), 8 U/µl Ampli Taq DNA polymerase (0.2 µl) and H₂O (36.9 µl), and PCR reaction was conducted in 25 cycles of 94°C for 1 min., 72°C for 1 min. and 72°C for 2 min.

### (2) Restriction enzyme analysis of mel-1a

The clones, which had been confirmed to have the objective molecular weight size inserted by colony PCR, was subjected to restriction enzyme analysis after recovery of the plasmid. Each of the clones was cultivated in an LB culture medium containing 50 µl/ml of ampicillin at 37°C overnight. A 1.5 ml portion of each culture broth was freed of the plasmid for recovery, and the remaining culture broth was admixed with an equal volume of 80 % glycerol, followed by storing at -20°C.

Added to a Mirotube were a solution (1 µl) each containing a combination of different restriction enzymes, (A) Xbal and HindIII, (b) Hind III and HifI and (c) BssHII, 10-fold diluted reaction buffer (Note 4) (1 µl) and plasmid DNA (8 µl), followed by reaction at 37°C for 2 hours. Each enzyme-digested product was subjected to electrophoresis, followed by staining with ethydium bromide and photographing under UV irradiation.
Note 4: As attached to each restriction enzyme.

mel-1a as amplified from the whole brain by semi-nested PCR was ligated to pCR3-Uni vector, followed by transfection into Top10F' competent cell. The resultant 67 colonies were examined by PCR, leading to the confirmation that 19 colonies had the vector inserted therein. The restriction-enzyme analysis of each colony is shown in Figs. 5 and 6, in which the sings (A), (B) and (C) designate the enzyme treatments with Xbal + HindIII, HindIII + HinfI, and BssHII, respectively, and the clones not having the objective molecular weight detected were illustrated in Fig. 6 with the sign "X", with clone Nos. 2, 19, 22, 31, 35, 46, 49, 53, 54, 56, 63 and 64 being confirmed to be normal by means of the restriction-enzyme analysis.

### (3) Sequencing of mel-1a DNA

As reagents, 5-fold diluted TACS buffer (Note 5) (4 µl), 2.5 mM dNTP mixture (1 µl), G Dye Terminator (1 µl), C Dye Terminator (2.1 µl), T Dye Terminator (1 µl), A Dye Terminator (1 µl), 13U/µl Ampli Tag DNA polymerase (0.5 µl), 5 µM sequencing primer (1 µl), plasmid DNA (3.5 µl) and SDDW (6 µl) were added to a Microtube, followed by 25 cycles of PCR reaction at 96°C for 30 sec., at 50°C for 15 sec. and at 60°C for 4 min. Note 5: Terminator Ammonium Terminator Cycle Sequencing buffer.

Added to the reaction solution were a one-tenth volume of 3M sodium acetate solution and a two-fold volume of 100% ethanol, and after cooling over an ice bath for 30 min., the centrifugation procedure was carreid out at 15,000 rpm for 15 min. The solution mixture was freed of the supernatant, then admixed with 80 % ethanol and centrifuged quickly to remove the supernatant, and the remaining water was removed by evaporation in a centrifuge evaporator.

The resultant residue was admixed with 3 µl of a loading solution (supplied by Nippon Gene Co. of Japan), followed by heating at 94°C for 2 min. and quick cooling over an ice bath. The resultant sample was applied to a DNA sequencer, and then electrophoresis was effected (Figs. 7 and 8).

As a result, all of the selected clones were observed to undergo mutation by replacing adenine with guanine at the 90th position. However, there was observed no placement in leucine of the encoding amino acid, with the silent base mutation occurring, and consequently, clone No. 2 (Figs. 7 and 8) were transfected into CHO cell to generate a transformed cell.

### Example 3: Preparation in large quantities of mel-1a/pCR3-Uni and analysis with a cytosensor.

(1) On the basis of the restriction enzyme analysis and sequencing results, the least mutated clone was selected. The selected Escherichia coli was cultivated in 1 liter of LB culture medium containing 50 µg/ml ampicillin at 37°C overnight, and then the cells were cooled over an ice bath for 10 min. and centrifuged at 3,000 rpm at 4°C. The precipitate was suspended in 10 ml of TES (50 mM Tris/HCl, pH 8.0, 25 % sucrose, 40 mM EDTA), and the suspension was admixed with 1 ml of 10 mg/ml lysozyme, followed by stirring. The reaction mixture was incubated at room temperature for 10 to 20 min., then admixed with 4 ml of Lysis Buffer (2 % Triton, 50 mM Tris/HCl, pH 8.0), furthermore incubated at room temperature for 30 min. and centrifuged at 3,000 rpm at 18°C for 30 min. The resultant supernatant was transferred into a tube, to which 0.95 g of cesium chloride and 0.1 ml of 10 mg/ml ethidium bromide were added for each ml of the supernatant, followed by stirring and centrifugation at 10,000 rpm at 18°C for 30 min.

The resultant supernatant alone was transferred into a ultra-centrifugation tube, followed by centrifugation at 90,000 rpm for 15 hours. Immediately after centrifugation, a band of DNA fraction was collected by use of a 20G needle and admixed with a phenol-chloroform (1:1) solution, and the resultant mixture was stirred thoroughly and centrifuged at 13,000 rpm for 5 min. The upper layer was removed in such a manner as the DNA phase in intermediate layer might not be discharged off.

The lower and intermediate layers were recovered and admixed with a two-fold volume of water and a six-volume of ethanol, followed by cooling at -20°C for several hours and centrifugation at 13,000 rpm for 5 min. The precipitate was dissolved in 12 ml of TENS (10 mM Tris/HCl, pH 7.5, 1 mM EDTA, 0.4M NaCl, 0.5 % SDS) buffer to effect treatment with phenol. The precipitate was dissolved in TE (10 mM Tris/HCl, pH 7.5, 1 mM EDTA), and another ethanol precipitation was carried out. The resultant precipitate was washed with 70 % ethanol and dissolved in 0.5 ml of TE buffer, and the solution was subjected to spectrophotometry, whereupon the concentration of mel-1a/pCR-Uni was determined from the measured absorbance.

### Reagents Used and Their Suppliers (indicated in parentheses):

Human fetal brain poly A+RNA (CLONTECH); human thalamus poly A+RNA (CLONTECH); human whole brain poly A+RNA (CLONTECH); Red-TO-Go T-Primed First-Strand Kit (Pharmacia Biotech); Ex taq DNA polymerase (TAKARA); Ampli-taq DNA polymerase (PERKIN-ELMER); Eukaryotic Ta Cloning Kit (Invitrogen); Gene Clean 11 (BIO 101); RPM Kit (Bio 101); HindIII (TAKARA); Xbal (TAKARA); HinfI (TAKARA); BssHII (TAKARA); Dye Terminator Cycle Sequencing Cor Kit (PERKIN-ELMER); SEAKEM GTG Agarose (FMC, Bio-Products), NUE SIEVE 3;1 Agarose (FMC Bio-Products); SOC medium (GIBCO BRC); Bacto LB BROT H, RENNOX (DIFCO); Bacto LB AGAR, RENNOX (DIFCO); Ampicillin (SIGMA); 20 mg/ml ethydium bromide solution (Nippon Gene).

### (2) Transfection of the plasmid (mel-1a/pCR3-Uni) into CHO cells

CHO cells (Dai-Nippon Pharmaceutical Co. of Japan) were cultivated in the DMEM medium (Nippon Seiyaku Co. of Japan) containing 10 % FBS (fetal bovine serum, Ma Bio) under the conditions of 5% CO₂/37°C. In order to transfect the recombinant plasmid (mel-1a/pCR3-Uni) by the calcium phosphate method, CHO cell in the logarithmic growth phase was seeded at a concentration of 5 x 10⁵ cells in a 100 mm dish for cultivation (IWAKI), and cultivated overnight.

mel-1a/pCR3-Un, after being mixed with th reagent of Kit (Stratagene), was added to a culture dish at a ratio of 10 to 30 µg/dish and cultivated overnight under the conditions of 37°C/3% CO₂, and the cells were washed twice with phosphate buffer (PBS, supplied by Nissui Seiyaku Co. of Japan) and cultivated additionally in the newly exchanged medium overnight under 37°C/3 % CO₂. The cells were washed once with pBS, treated with trypsin (MA Bio), then diluted with PBS to 10 to 30 fold in cell concentration, seeded in a 6-well plate (IWAKI) and cultivated overnight. The cells were washed once with PBS and cultivated overnight in the newly exchanged DMEM medium containing 800 µg/ml of C418 (Sigma) and 10% of FBS. Thereafter, the medium was exchanged very three days, and cultivation was continued in the 6-well plate until the cells not having transfected with the plasmid (mel-1a/pCR3-Uni) were entirely dead. The resultant transfected cells was deposited with Institute of Bio-Engineering Industry and Technology, The Agency of Industry and Technology, MITI of Japan (The Deposition No. FERM P-16224).

### Reagents Used and Their Suppliers (indicated in parentheses):

Ampicillin (SIGMA); Bacto LB BROTH, RENNOX (DIFCO); tris (hydroxymethyl)aminomethane (Wako Pure Chemical); HCl (Wako Pure chemical); sucrose (Wako Pure Chemical); EDTA (Wako Pure Chemical); lysozyme (Wako Pure Chemical(; Triton X 100 (Wako Pure Chemical); phenol/chloroform (GIBCO); CHO cell (Dai-Nippon Pharmaceuticals), DMEM medium (Nissui Seiyaku); FBS (MA Bio); Genestein (SIGMA); PBS(-) (Nissui Seiyaku); gentamycin (SIGMA); MAMMALIAN TRANSFECTION KIT (TOYOBO).

### (3) Analysis with a cytosensor

### [Preparation of cell capsule]

mel-1a/CHO cells were cultivated in advance in the DMEM medium containing 800 µg/ml G418 and 10% FBS, and mel-1a/CHO cells in the logarithmic growth phase were washed with PBS, treated with trypsin and diluted with the same medium to a concentration of 2.5 x 10⁵/ml. 1 ml of cell suspension was seeded in each cell cup for cytosensor, and the cell cups were filled on the outside with 2 ml of the medium and then left on standing overnight under the conditions of 5% CO₂/37°C.

While paying attention not to cause air bubbles to mingle, a spacer was disposed on the bottom of each cell cup with use of sterile tweezers on the following day. When air bubbles were below the spacer, they were to be removed with a pipette. A capsule insert was placed in each cup, while preventing air bubbles from mingling, and the medium in the 1 ml portion was added from the top to thereby bury the cup in the medium, whereby it was to be ascertained that no air bubble had been drained or mingled into the spacer and insert.

### [Preparation for the measuring medium]

Powdered low buffer RPM1 1640 medium (MDC) was dissolved in 1 liter of distilled water for tissue culture (Cosmo Bio), and the solution was adjusted to pH 7.4 and then suction-filtered through a 0.45 µm filter.

### [Preparation for the cytosensor system]

The Cyto software package of a personal computer was started up. A measuring medium was set in a cytosensor, and fluid feeding system was switched for the measuring medium. The fluid inside the sensor chamber was aspirated out, and the measuring medium was placed in the 1.5 ml portion in to each cell, where a capsule cell prepared in advance was disposed while preventing air bubbles from mingling. The sensor chamber was connected to a cytosensor without causing air bubbles to mingle.

### [Measurement of the metabolic rate with melatonin and homologs]

After initiation of the data collection, the measuring medium continued to be fluid-fed until the metabolic rate was stabilized, and a required amount each of melatonin and its homologs were added to the measuring medium to make the samples. Each of the prepared samples wag set on the cytosensor under the conditions where the metabolic rate was stabilized. Measurement with the cytosensor was performed by switching the fluid feeding from the measuring medium to the sample solution to stimulate the cells, then putting the fluid-feeding back to the measuring medium and measuring a change caused in metabolic rate of the cells, whereby stimulation with the sample was effected for 2 min. and subsequently the measuring medium continued to be fluid-fed until the metabolic rate was returned to the level determined prior to stimulation with the sample. Normally, it took about 40 min. for the metabolic rate to return to the status prior to stimulation with the sample, once stimulation was provided with melatonin. Accordingly, stimulation with the sample was given at a regular interval of 40 min.

The cytosensor was designed to detect the release outside the cell of hydrogen ions, which is understood to reflect the metabolic activity of the cell, as a rate of change in extracellular hydrogen ion concentration. Upon binding of a ligand to the receptor expressed on the cell surface, intracellular signal transduction takes place, resulting in increased cellular metabolic activity to thereby release hydrogen ions outside the cell.

When melatonin was added in quantities ranging from 1 pM to 100 nM to the CHO cell having the mel-1a receptor expressed therein, the cellular metabolic activity was observed to rise in the dose-dependent manner at above 100 pM in the concentrations of melatonin (Fig. 9).

An increase in cellular metabolic activity by melatonin reached the maximum simultaneously with the addition of melatonin and decreased down to the level prior to the melatonin addition over the period of about 40 min. In Fig. 10 is shown the rate of change in the cellular metabolic activity determined in the case of addition of melatonin at different concentrations at the regular interval of 40 min. When the maxima of the cellular metabolic activity were plotted against the melatonin added at different concentrations, there was obtained a dose-dependent saturation curve, with the linear rise and saturation being noted at above 100 pM and 1 nM in the concentrations of melatonin, respectively (Fig. 10).

In addition to melatonin, its homologs tryptophan, methoxytryptophan, methoxytryptamine, hydroxytryptamine and acetylserotonin were employed to test the responsiveness of the melatonin receptor mel-1a with a cytosensor (Fig. 11).

As a result, it is evident that the melatonin expressed cell of the present invention responds specifically to melatonin alone, whereas it does not bring about any increase in metabolic activity with any of its homologs, providing a highly specific analysis system with enhanced degrees of sensitivity (Fig. 11).

### Reagents Used and Their Suppliers (indicated in parentheses):

Melatonin (Tokyo Kasei Kogyo); serotonin (SIGMA); N-acetylserotonin (SIGMA); 5-hydroxytryptamin (SIGMA); cytosensor microphysiometer (NMD); cytosensor capsule kit (NMD); cytosensor-degassing membrane (NMD); cytosensor sterilizing kit (NMD); RPMI medium for cytosensor (NMD).

## Claims

1. A material for screening a substance showing affinity for a melatonin receptor protein, which comprises an animal cell containing an expression plasmid for the gene encoding a human melatonin receptor protein and having the protein expressed therein.

2. A screening material as claimed in claim 1, wherein the gene encoding a melatonin receptor protein is a membrane receptor gene or a nuclear receptor gene.

3. A screening material as claimed in claim 1, wherein the animal cell is the CHO cell.

4. An animal cell possessing an expression plasmid for the gene encoding a human melatonin receptor protein, which animal cell has a recombinant human melatonin receptor protein expressed therein.

5. An animal cell as claimed in claim 4, wherein the human melatonin receptor protein has a gene encoding a derivative protein generated by subjecting its amino acids or peptides to replacement, deletion or addition to such as extent as may retain substantially identical activity and has the said gene expressed therein.

6. An animal cell as claimed in claim 4, wherein the said animal cell is the CHO cell.

7. An animal cell as claimed in claim 4, wherein the said animal cell is Mel la/CHO.

8. An expression plasmid for a gene encoding a human melatonin receptor protein or for a gene encoding a derivative of a human melatonin receptor protein.

9. A method for screening a hormone showing affinity for a human melatonin receptor and its agonist or antagonist which comprises using an expression cell for a human melatonin receptor protein to thereby measure a change in metabolic activity of the cell.

10. A recombinant human melatonin receptor protein produced by cultivating an animal cell containing a gene encoding a human melatonin receptor protein and being capable of expressing a recombinant human melatonin receptor protein exhibiting substantially the same, homogeneous activity as the natural human melatonin receptor protein.

11. A polyclonal antibody and monoclonal antibody against a recombinant human melatonin receptor protein.


SEQUENCE LISTING

(1) GENERAL INFORMATION:

APPLICANT:
(A) NAME: JCR Pharmaceuticals Co., Ltd.
(B) STREET: 3-19, Kasuga-cho, Ashiya
(C) CITY: Hyogo 659-0021
(E) COUNTRY: Japan
(F) POSTAL CODE (ZIP): .

TITLE OF INVENTION:
Melatonin-Receptor Expression Cells
and Their Uses

NUMBER OF SEQUENCES: 7

COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PADAT Sequenzmodul, Version 1.0

CURRENT APPLICATION DATA:
(A) APPLICATION NUMBER: EP 98.111352.5

(2) INFORMATION FOR SEQ ID NO: 1:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 1:

ATGGCCCTGC GGCCGGGACG CGAAC 25

(2) INFORMATION FOR SEQ ID NO: 2:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 2:

GACCATGCAG GGCAACGGCA GCGC 24

(2) INFORMATION FOR SEQ ID NO: 3:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 3:

TTAAACGGAG TCCACCTTTA CTAC 24

(2) INFORMATION FOR SEQ ID NO: 4:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 4:

TATCTGCACT GCCAAGTCAG TGGGTTCCTG 30

(2) INFORMATION FOR SEQ ID NO: 5:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 5:

GGTTCTCCAG GTCAGACAGA GGGTGAAACC 30

(2) INFORMATION FOR SEQ ID NO: 6:

(i) SEQUENCE CHARACTERISTICS:
(A) LENGTH: 1053 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 6:

ATGCAGGGCA ACGGCAGCGC GCTGCCCAAC GCCTCCCAGC CCGTGCTCCG CGGGGACGGC 60

GCGCGGCCCT CGTGGCTGGC GTCCGCCCTG GCCTGCGTCC TCATCTTCAC CATCGTGGTG 120

GACATCCTGG GCAACCTCCT GGTCATCCTG TCGGTGTATC GGAACAAGAA GCTCAGGAAC 180

GCAGGAAACA TCTTTGTGGT GAGCTTAGCG GTGGCAGACC TGGTGGTGGC CATTTATCCG 240

TACCCGTTGG TGCTGATGTC GATATTTAAC AACGGGTGGA ACCTGGGCTA TCTGCACTGC 300

CAAGTCAGTG GGTTCCTGAT GGGCCTGAGC GTCATCGGCT CCATATTCAA CATCACCGGC 360

ATCGCCATCA ACCGCTACTG CTACATCTGC CACAGTCTCA AGTACGACAA ACTGTACAGC 420

AGCAAGAACT CCCTCTGCTA CGTGCTCCTC ATATGGCTCC TGACGCTGGC GGCCGTCCTG 480

CCCAACCTCC GTGCAGGGAC TCTCCAGTAC GACCCGAGGA TCTACTCGTG CACCTTCGCC 540

CAGTCCGTCA GCTCCGCCTA CACCATCGCC GTGGTGGTTT TCCACTTCCT CGTCCCCATG 600

ATCATAGTCA TCTTCTGTTA CCTGAGAATA TGGATCCTGG TTCTCCAGGT CAGACAGAGG 660

GTGAAACCTG ACCGCAAACC CAAACTGAAA CCACAGGACT TCAGGAATTT TGTCACCATG 720

TTTGTGGTTT TTGTCCTCTT TGCCATTTGC TGGGCTCCTC TGAACTTCAT TGGCCTGGCC 780

GTGGCCTCTG ACCCCGCCAG CATGGTGCCT AGGATCCCAG AGTGGCTGTT TGTGGCCAGT 840

TACTACATGG CGTATTTCAA CAGCTGCCTC AATGCCATTA TATACGGGCT ACTGAACCAA 900

AATTTCAGGA AGGAATACAG GAGAATTATA GTCTCGCTCT GTACAGCCAG GGTGTTCTTT 960

GTGGACAGCT CTAACGACGT GGCCGATAGG GTTAAATGGA AACCGTCTCC ACTGATGACC 1020

AACAATAATG TAGTAAAGGT GGACTCCGTT TAA 1053

(2) INFORMATION FOR SEQ ID NO: 7:

(i) SEQUENCE CHARACTERISTICS:
(A) LENGTH: 350 amino acids
(B) TYPE: amino acid
(C) STRANDEDNESS: single
(D) TOPOLOGY: linear

(ii) MOLECULE TYPE: Protein

(v) FRAGMENT TYPE: internal

(xi) SEQUENCE DESCRIPTION: SEQ ID NO: 7:

Met Gln Gly Asn Gly Ser Ala Leu Pro Asn Ala Ser Gln Pro Val Leu
1               5                   10                  15

Arg Gly Asp Gly Ala Arg Pro Ser Trp Leu Ala Ser Ala Leu Ala Cys
            20                  25                  30

Val Leu Ile Phe Thr Ile Val Val Asp Ile Leu Gly Asn Leu Leu Val
        35                  40                  45

Ile Leu Ser Val Tyr Arg Asn Lys Lys Leu Arg Asn Ala Gly Asn Ile
    50                  55                  60

Phe Val Val Ser Leu Ala Val Ala Asp Leu Val Val Ala Ile Tyr Pro
65                  70                  75                  80

Tyr Pro Leu Val Leu Met Ser Ile Phe Asn Asn Gly Trp Asn Leu Gly
                85                  90                  95

Tyr Leu His Cys Gln Val Ser Gly Phe Leu Met Gly Leu Ser Val Ile
            100                 105                 110

Gly Ser Ile Phe Asn Ile Thr Gly Ile Ala Ile Asn Arg Tyr Cys Tyr
        115                 120                 125

Ile Cys His Ser Leu Lys Tyr Asp Lys Leu Tyr Ser Ser Lys Asn Ser
    130                 135                 140

Leu Cys Tyr Val Leu Leu Ile Trp Leu Leu Thr Leu Ala Ala Val Leu
145                 150                 155                 160

Pro Asn Leu Arg Ala Gly Thr Leu Gln Tyr Asp Pro Arg Ile Tyr Ser
                165                 170                 175

Cys Thr Phe Ala Gln Ser Val Ser Ser Ala Tyr Thr Ile Ala Val Val
            180                 185                 190

Val Phe His Phe Leu Val Pro Met Ile Ile Val Ile Phe Cys Tyr Leu
        195                 200                 205

Arg Ile Trp Ile Leu Val Leu Gln Val Arg Gln Arg Val Lys Pro Asp
    210                 215                 220

Arg Lys Pro Lys Leu Lys Pro Gln Asp Phe Arg Asn Phe Val Thr Met
225                 230                 235                 240

Phe Val Val Phe Val Leu Phe Ala Phe Cys Trp Ala Pro Leu Asn Phe
                245                 250                 255

Ile Gly Leu Ala Val Ala Ser Asp Pro Ala Ser Met Val Pro Arg Ile
            260                 265                 270

Pro Gln Trp Leu Phe Val Ala Ser Tyr Tyr Met Ala Tyr Phe Asn Ser
        275                 280                 285

Cys Leu Asn Ala Ile Ile Tyr Gly Leu Leu Asn Gln Asn Phe Arg Lys
    290                 295                 300

Glu Tyr Arg Arg Ile Ile Val Ser Leu Cys Thr Ala Arg Val Phe Phe
305                 310                 315                 320

Val Asp Ser Ser Asn Asp Val Ala Asp Arg Val Lys Trp Lys Pro Ser
                325                 330                 335

Pro Leu Met Thr Asn Asn Asn Val Val Lys Val Asp Ser Val
            340                 345                 350